(19) Europäisches Patentamt
European Patent Office
Office européen des brevets

(11) Publication number: 0 022 578 B1

(12) **EUROPEAN PATENT SPECIFICATION**

(45) Date of publication of patent specification: 29.06.88

(21) Application number: 80104029.6

(22) Date of filing: 11.07.80

(51) Int. Cl.4: A 61 K 31/415, A 61 K 31/44, C 07 D 231/06, C 07 D 401/04 // C07C109/04, C07C79/12, C07C87/66, C07C87/68

(54) **Pharmaceutical compositions containing 3-Amino-pyrazoline derivatives.**

(30) Priority: 13.07.79 US 57362

(43) Date of publication of application: 21.01.81 Bulletin 81/03

(45) Publication of the grant of the patent: 29.06.88 Bulletin 88/26

(84) Designated Contracting States: BE CH DE FR GB LI LU NL SE

(56) References cited:
DE-A-2 727 706
GB-A-1 324 687

PROSTAGLANDINS, vol. 18, no. 2, August 1978, Los Altos, California, US. J.J. ADCOCK et al.: "The mechanism of enhancement by fatty acid hydroperoxides of anaphylactic mediator release", pages 179-187

CHEMICAL ABSTRACTS, vol. 54, no. 2, January 25, 1960, abstract 1501b-f. A.N. KOST et al.: "Reactions of hydrazine derivatives. XXII. 3-Amino-arylpyrazolines and their saliclidene derivatives"

(73) Proprietor: THE WELLCOME FOUNDATION LIMITED
183-193 Euston Road
London NW1 2BP (GB)

(72) Inventor: Challand, Stanley Richard
40 Braeside
Beckenham Kent (GB)
Inventor: Copp, Frederick Charles
"Rotherwood" 32 Stanley Avenue
Beckenham Kent (GB)
Inventor: Denyer, Clive Vincent
73 Rowan Crescent Streatham Vale
London S.W.16 (GB)
Inventor: Eakins, Kenneth Ernest
26 Kelsey Way
Beckenham Kent (GB)
Inventor: Walker, John Michael Graham
14 Hayes Way
Beckenham Kent (GB)
Inventor: Whittaker, Norman
37 Brabourne Rise
Beckenham Kent (GB)
Inventor: Caldwell, Albert Gordon
119 Gates Green Road
West Wickham Kent (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 022 578 B1

(74) Representative: **Sandmair, Kurt, Dr. et al**
**Patentanwälte Schwabe, Sandmair, Marx**
**Stuntzstrasse 16**
**D-8000 München 80 (DE)**

**Description**

This invention relates to pharmaceutical formulations adapted to only topical, to the exclusion of oral and injectable, administration and comprising heterocyclic compounds.

The heterocyclic compounds referred to herein are of formula (I):

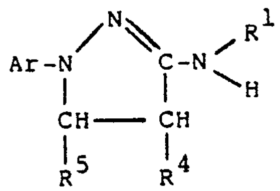

(I)

wherein,

Ar is selected from pyridyl or phenyl, each of which may be optionally substituted in one or two positions in the ring by the same or a different substituent, said substituent being selected from trifluoromethyl, fluoro, chloro, bromo and iodo;

$R^1$ is selected from hydrogen and acyl having from 1 to 4 carbons atoms; and

$R^4$ and $R^5$ are the same or different and each is selected from hydrogen and alkyl having from 1 to 4 carbon atoms;

and acid addition salts thereof.

The heterocyclic compounds of formula (I) are members of a class of compounds encompassed within UK patent specification No. 1 324 687 and alleged to have anti-spasmodic activity. Certain of the heterocyclic compounds of formula (I) are known as chemical intermediates in UK patent specification Nos. 1 515 500, 679678 ad 679677. In Biochem. Pharmacol. (1979), *28*, 1959—1961 it is disclosed that one such heterocyclic compound of formula (I), namely 3-amino-1-(*m*-trifluoromethylphenyl)pyrazol-2-ine, has been shown to have anti-inflammatory activity in the rat when administered orally in the carrageenin-induced oedema assay. Related 1-alkyl-3-amino-4-arylpyrazol-2-ines are disclosed in UK patent specification No. 1 297 035 as having anti-inflammatory, analgesic or antipyretic activity.

It has now been found that the heterocyclic compounds of formula (I) exhibit a potent anti-inflammatory action in mammals when administered topically and that such activity can be achieved at doses which do not provide the side-effects associated either with their systemic (e.g. oral) administration or the administration of certain other topical anti-inflammatories. In comparison with the compounds disclosed in UK Patent No. 1 294 035, the heterocyclic compounds of formula (I) exhibit more potent anti-inflammatory activity and some heterocyclic compounds of formula (I) are more selective in inhibiting the lipoxygenase pathway of arachidonic acid metabolism than 3-amino-1-(*m*-trifluoromethylphenyl)pyrazol-2-ine. The anti-inflammatory activity of the heterocyclic compounds of formula (I) on topical administration is surprising in view of the teaching of the antispasmodic activity claimed for them in UK Patent No. 1 324 687. None of the antispasmodic agents described in The Pharmacological Basis of Therapeutics, 5th Edition, by Goodman and Gilman, pages 514 to 532 Macmillan, (1975) have anti-inflammatory activity. It is even more surprising since it has been found that the heterocyclic compounds referred to herein have aspirin-like (i.e. peripheral) not central analgesic activity.

Preferred heterocyclic compounds of formula (I) are those wherein, Ar is selected from 3-substituted phenyl, 4-substituted phenyl, 3,4-disubstituted phenyl and 5-substituted pyridyl; $R^1$ is selected from hydrogen and acetyl; and $R^4$ and $R^5$ are the same or different and each is selected from hydrogen and methyl.

Where Ar is substituted phenyl, it is preferred that Ar is 3- or 4-trifluoromethylphenyl or phenyl substituted by one or more of fluoro, chloro or bromo in the 3-, 4- or 3,4- positions.

More preferred heterocyclic compounds of formula (I) are those wherein Ar is selected from phenyl substituted by one or two substituent(s) selected from 4-fluoro, 4-chloro, 4-bromo and 3-trifluoromethyl, and pyridyl substituted by one substituent selected from 5-chloro, 5-bromo and 5-iodo, such as 3-amino-1-(*m*-trifluoromethylphenyl)-2-pyrazoline which is particularly preferred.

It is also preferred that in the compounds of formula (I), $R^1$ is hydrogen.

Especially preferred heterocyclic compounds of formula (I) are those wherein, one of $R^4$ and $R^5$ is methyl, such as: 3-amino-1-(*p*-fluorophenyl)-4-methyl-2-pyrazoline; and 3-amino-1-(*p*-fluorophenyl)-5-methyl-2-pyrazoline.

While used in medicine, the acid addition salts of a heterocyclic compound of formula (I) should be both pharmacologically and pharmaceutically acceptable acid addition salts, but non-acceptable salts may be conveniently used to prepare the bases of such acceptable salts. Acceptable salts may be derived from organic acids, particularly dicarboxylic acids. Such pharmacologically and pharmaceutically acceptable salts include those prepared from the following acids: hydrochloric, hydrobromic, sulphuric, nitric, phosphoric, oxalic, fumaric, maleic, glycolic, salicyclic, succinic, toluene-*p*-sulphonic, tartaric, acetic, citric, methane-sulphonic, formic, benzoic, malonic, naphthalene-2-sulphonic and benzene-sulphonic.

The heterocyclic compounds of formula (I) may be prepared by any method known in the art for the preparation of compounds of analogous structure.

Suitable methods of preparing the compounds of formula (I) wherein $R^1$ is hydrogen include reacting a compound of formula (II):

$$Ar—NH.NHH_2 \qquad (II)$$

with an acrylonitrile of formula (III):

$$R^5CH=C(R^4).CN \qquad (III)$$

wherein Ar, $R^4$ and $R^5$ are as defined in formula (I) hereinabove.

The reaction may be carried out by standard techniques known in the art, conveniently in a polar solvent such as alkanol for example ethanol, in the presence of a base such as sodium in ethanol or sodium hydroxide, with or without heating or cooling and optionally in a nitrogen atmosphere.

Suitable methods of preparing the compounds of formula (I) wherein $R^1$ is acyl as defined hereinabove include acylating a compound of formula (I) wherein $R^1$ is hydrogen and Ar, $R^4$ and $R^5$ are as defined in formula (I) hereinbefore. The acylation may be effected by standard methods which include reaction with the appropriate acid anhydride for example acetic anhydride, acid halide for example acetyl chloride, or mixed anhydride such as acetic anhydride in formic acid. The reaction may be effected in an unreactive solvent such as chloroform and at an elevated temperature (60—80°) where necessary; or without a solvent when lower temperatures are preferable. Acylation may also be effected by reaction with butyl lithium followed by reaction with the appropriate carboxylic acid ester, for example ethyl butyrate.

Alternatively, the heterocyclic compounds of formula (I) wherein Ar is phenyl or substituted phenyl may be prepared according to the method of G. F. Duffin and J. D. Kendall in J. Chem. Soc. (1954), 411—412. For example by reduction of a compound of formula (IV):

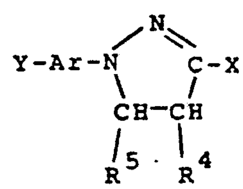

(IV)

wherein one of X or Y is N=NAr and when X is N=NAr, Y is hydrogen and when Y is N=NAr, X is $NHR^1$; and Ar, $R^4$, $R^5$ and $R^1$ are as defined, with a reducing agent such as zinc and a carboxylic acid such as acetic acid or stannous chloride and a mineral acid such as hydrochloric acid in a unreactive polar solvent such as an alkanol, for example, ethanol.

Alternatively, the heterocyclic compounds of formula (I) may be prepared according to the methods described in UK patent specification No. 1 324 687 such as mild hydrolysis of a 1,2,4-oxadiazole of formula (V) with, for example, sodium ethylate:

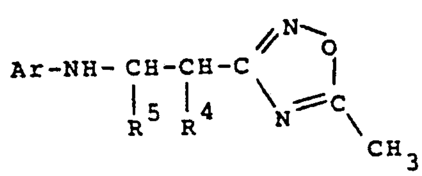

(V)

wherein Ar, $R^4$ and $R^5$ are as defined in formula (I) to produce a compound of formula (I) wherein $R^1$ is acyl. Further hydrolysis using, for example, sodium hydroxide would produce the compound wherein $R^1$ is hydrogen. Or they may be prepared by reacting a compound of formula (VI)

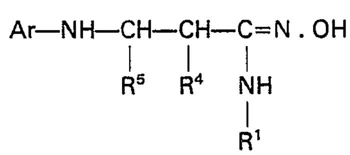

(IV)

with a reagent such as toluenesulphonyl chloride in a solvent such as pyridine.

The heterocyclic compound of formula (I) prepared by any of these methods may be optionally inter-converted to any other desired compound of formula (I). For example, a compound of formula (I) wherein $R^1$ is acyl may be converted to the corresponding compound wherein $R^1$ is hydrogen or *vice versa*.

The heterocyclic compounds of formula (I) may be used in the treatment or prophylaxis of inflammation in a mammal, including man, and may be administered topically in the relief of rheumatoid arthritis, rheumatoid spondylitis, osteoarthritis, gouty arthritis, adjuvant arthritis and other arthritic conditions, inflamed joints, eczema, psoriasis or other inflammatory skin conditions such as sunburn;

inflammatory eye conditions including conjunctivitis; pyresis, pain and other conditions associated with inflammation.

By topical administration is meant non-systemic administration and includes the application of a heterocyclic compound of formula (I) externally to the epidermis, to the buccal cavity and instillation of such a compound into the ear, eye and nose, and where the compound does not significantly enter the blood stream.

By systemic administration is meant oral, intravenous, intraperitoneal and intramuscular administration.

The amount required of a compound of formula (I) (hereinafter referred to as the active ingredient) for therapeutic effect will, of course, vary with the compound chosen, the nature and severity of the inflammatory condition and the mammal undergoing treatment and is ultimately at the discretion of the physician. A suitable anti-inflammatory dose of an active ingredient is 1 ng to 500 mg of base per kilogram bodyweight, the most preferred dosage being 10 ng to 5 mg/kg of mammal bodyweight, for example 0.1 to 2 µg/kg administered at four-hourly intervals, as required. For application to the skin, from 1 µg to several mg of the active ingredient may be applied per application, preferably from 10 to 100 µg per application.

In the pharmaceutical formulations according to the present invention, the active ingredient may comprise from 0.001% to 10% by weight of the formulation; conveniently, not in excess of 5% w/w; and preferably from 0.1% to 196 w/w of the formulation. The balance of the formulation comprises one or more acceptable carriers therefor and optionally other therapeutic ingredients. The carrier(s) must be 'acceptable' in the sense of being compatible with the other ingredients of the formulation and not deleterious to the recipient therefor.

Formulations adapted for topical administration as hereinbefore defined include liquid or semi-liquid preparations suitable for penetration through the skin to the site of inflammation such as liniments, lotions, creams, ointments or pastes, and drops adapted for administration to the eye, ear or nose.

Drops according to the present invention may comprise sterile aqueous or oily solutions or suspensions and may be prepared by dissolving the active ingredient in a suitable aqueous solution of a bactericidal and/or fungicidal agent and/or any other suitable preservative, and preferably including a surface active agent. The resulting solution may then be clarified by filtration, transferred to a suitable container which is then sealed and sterilized by autoclaving or maintaining at 98—100°C for half an hour. Alternatively, the solution may be sterilised by filtration and transferred to the container by an aseptic technique. Examples of bactericidal and fungicidal agents suitable for inclusion in the drops are phenylmercuric nitrate or acetate (0.002%), benzalkonium chloride (0.01%) and chlorhexidine acetate (0.01%). Suitable solvents for the preparation of an oily solution include glycerol, diluted alcohol and propylene glycol.

Lotions according to the present invention include those adapted for application to the skin or eye. An eye lotion may comprise a sterile aqueous solution optionally containing a bactericide and may be prepared by methods similar to those for the preparation of drops. Lotions or liniments for application to the skin may also include an agent to hasten drying and to cool the skin, such as an alcohol or acetone, and/or a moisturiser such as glycerol or an oil such as castor oil or arachis oil.

Creams, ointments or pastes according to the present invention are semi-solid formulations of the active ingredient for external application. They may be made by mixing the active ingredient in finely-divided or powdered form alone or in solution or suspension in an aqueous or non-aqueous fluid, with the aid of suitable machinery, with a greasy or non-greasy basis. The basis may comprise hydrocarbons such as hard, soft or liquid paraffin, glycerol, beeswax, a metallic soap; a mucilage, an oil of natural origin such as almond, corn, arachis, castor or olive oil, wool fat or its derivatives, or a fatty acid such as stearic or oleic acid together with an alcohol such as propylene glycol or macrogols. The adapted formulation may incorporate any suitable surface active agent such as an anionic, cationic or non-ionic surfactant such as sorbitan esters or polyoxyethylene derivatives thereof. Suspending agents such as natural gums, cellulose derivatives or inorganic materials such as silicaceous silicas; and other ingredients such as Janolin may also be included.

The following Examples are provided by way of an illustration of the present invention and should in no way be construed as a limitation thereof. All temperatures indicated are in degrees Celsius.

Reference Example 1

Preparation of 3-Amino-1-m-trifluoromethylphenylpyrazol-2-ine

A. *Preparation of m-Trifluoromethylphenyl-hydrazine*

*m*-Aminobenzotrifluoride (48.34 g: 0.3 mole) was cooled and treated with concentrated hydrochloric acid (137 ml) added dropwise. The paste was stirred at 0° and a solution of sodium nitrite (19.1 g) in water (137 ml) was added at 0—5° with stirring. An almost clear solution was obtained. Then a solution of stannous chloride dihydrate (191.3 g) in concentrated hydrochloric acid (137 ml) was added dropwise with stirring at 0—10°.

Then the solid tin complex was filtered off. The solid was then added portionwise to ice-cold 10*N*-sodium hydroxide (2 l) and the oil collected into dichloromethane.

The dichloromethane solution was dried over anhydrous sodium sulphate, filtered, evaporated to dryness and distilled *in vacuo* to yield the title compound (m.p. 83—108° . 8 mm.).

B. *Preparation of 3-Amino-1-m-trifluoromethylphenylpyrazol-2-ine*

Sodium (0.50 g) was dissolved in absolute ethanol (25 ml). *m*-Trifluoromethylphenyl-hydrazine obtained in Example 1A (17.6 g: 0.1 mole) was then added dropwise with stirring to the clear solution. A pink colour developed. The solution was cooled in ice and acrylonitrile added (6.4 ml) dropwise. The clear pink solution was then stirred under reflux for 6 hours to give a crystalline solid on cooling. The solid was filtered off. On recrystallisation from cyclohexane (*ca* 400 ml) m.p. of the title compound was 107—108° (Mettler m.p. 109.1°: started 90° @ 20/min) after drying at 0.05 mm/½ hour.

Analysis $C_{10}H_{10}F_3N$

| | | | | | |
|---|---|---|---|---|---|
| Found: | C, 52,69 | : | H, 4,47 | : | N, 18,27 |
| Required: | C, 52,40 | : | H, 4,40 | : | N, 18,33 |

Examples 2—16

In accordance with the method described in Example 1 there were prepared the following:

| | |
|---|---|
| Reference Example 2: | 3-Amino-1-p-bromophenyl-2-pyrazoline m.p. 123—125° |
| Example 3: | 3-Amino-1-(3,4-dichlorophenyl)pyrazol-2-ine m.p. 184—185° (efferv.) |
| Reference Example 4: | 3-Amino-1-m-chlorophenyl-2-pyrazoline m.p. 129—130° (decomp.) |
| Reference Example 5: | 3-Amino-1-o-chlorophenyl-2-pyrazoline m.p. 104—105° |
| Reference Example 6: | 3-Amino-1-p-chlorophenyl-2-pyrazoline m.p. 149—151° |
| Reference Example 7: | 3-Amino-1-phenyl-2-pyrazoline m.p. 164—166° |
| Example 8: | 3-Amino-1-(4-chloro-3-trifluoromethyl)-phenyl-2-pyrazoline m.p. 146—147° |
| Reference Example 9: | 3-Amino-1-p-fluorophenyl-2-pyrazoline m.p. 114—115° |
| Example 10: | 3-Amino-1-p-fluorophenyl-4-methyl-2-pyrazoline m.p. 121—123° |
| Reference Example 11: | 3-Amino-5-methyl-1-phenyl-2-pyrazoline m.p. 101—103° |
| Example 12: | 3-Amino-1-m-chlorophenyl-4-methyl-2-pyrazoline m.p. 84—85° |
| Example 13: | 3-Amino-1-p-chlorophenyl-5-ethyl-2-pyrazoline m.p. 112—114° |
| Example 14: | 3-Amino-1-p-fluorophenyl-5-methyl-2-pyrazoline m.p. 133—135° |
| Reference Example 15: | 3-Amino-4-methyl-1-phenyl-2-pyrazoline m.p. 81—83° |
| Example 16: | 3-Amino-1-p-chlorophenyl-4-methyl-2-pyrazoline m.p. 113—115° |

Example 17

Preparation of 3-Amino-1-m-trifluoromethylphenylpyrazol-2-ine hydrochloride

3-Amino-1-m-trifluoromethylphenylpyrazol-2-ine obtained in Example 1 (4,48 g: 0,02 mole) was dissolved in N-hydrochloric acid (20 ml). The pale orange-coloured solution was then evaporated to dryness *in vacuo*. The solid residue was dissolved in ethanol containing 5% methanol and re-evaporated to dryness *in vacuo*, this process was repeated 3 times. The solid was dried at 0,1 mm at room temperature for 30 minutes. Recrystallisation from ethanol containing 5% methanol/ether gave the title compound m.p. 192—193°C.

Analysis: $C_{10}H_{11}F_3ClN_3$

| | | | | | |
|---|---|---|---|---|---|
| Found: | C, 45,04 | : | H, 4,06 | : | N, 15,8 |
| Required: | C, 45,21 | : | H, 4,17 | : | N, 15,81 |

Examples 18—19

In accordance with the method described in Example 17 there were prepared the following:

Example 18: 3-Amino-1-m-chlorophenyl-5-methyl-2-pyrazoline toluene-p-sulphonate m.p. 170—171°
Example 19: 3-Amino-1-p-chlorophenyl-5-methyl-2-pyrazoline hydrochloride m.p. 182—184°

Example 20

Preparation of 3-Amino-1-(2-pyridyl)-2-pyrazoline

2-Hydrazinopyridine (0.545 g) was added to a solution of sodium (50 mg; 0.43 atoms) in dry absolute ethanol (2 ml) in a nitrogen atmosphere. Acrylonitrile (0.265 g; 0.32 ml; 1 mol) was added dropwise and the resulting solution heated for 4 hours on the steam bath. The reaction mixture was cooled, giving some crystals, and treated with water (10 ml) to give a suspension of a solid. The solid was collected and washed with water. Recrystallisation from ethanol (*ca* 5 ml) gave the title compound m.p. 168°—169.5°. Thin Layer Chromatography: On $Al_2O_3$ in $CHCl_3$ gave a single spot Rf 0.18.

Examples 21 and 22

In accordance with the method described in Example 25 there were prepared the following:

Example 21: 3-amino-1-(4-pyridyl)-2-pyrazoline m.p. 286—287° (decomp.)
Example 22: 3-amino-1-(3-pyridyl)-2-pyrazoline m.p. 164—166°

Example 23

Preparation of 3-Amino-1-(2-pyridyl)-2-pyrazoline hydrobromide

3-Amino-1-(2-pyridyl)-2-pyrazoline obtained in Example 20 (100 mg) was taken up in ethanol containing 5% methanol (1 ml) and treated with excess concentrated aqueous hydrogen bromide. The resulting suspension of crystals was diluted with diethyl ether (*ca.* 8 ml) and filtered, and the crystals washed with diethyl ether: 5% methanol in ethanol (50:50). Concentration of the filtrate and treatment with diethyl ether gave more crystals which were discarded.

The pure title compound (99 mg) dissolved in water (1 ml) was treated with excess concentrated aqueous ammonia and set aside to crystallise at 0°; some concentrated aqueous potassium hydroxide was then added and after 10 minutes the pale buff crystals were collected and washed with water m.p. 168°—169.5°.

Reference Example 24

Preparation of 3-Acetylamino-1-(p-chlorophenyl)-pyrazol-2-ine

3-Amino-1-(*p*-chlorophenyl)pyrazol-2-ine (5 g) was added to acetic anhydride (4.0 ml) at room temperature. On stirring with a glass rod the mixture became warm and afforded a dark red liquid. The mixture was taken up in dichloromethane (100 ml) washed (water, 100 ml; saturated sodium bicarbonate, 100 ml; water, 100 ml), dried over sodium sulphate and the solvent removed *in vacuo* to afford the title compound (m.p. 171—173°).

Example 25

Preparation of 3-Butyramido-1-(3-chlorophenyl)-2-pyrazoline

In accordance with the method described in Example 23 there was prepared the title compound (m.p. 168—169°).

Reference Example 26

Preparation of 3-acetamido-4-methyl-1-phenyl-2-pyrazoline

3-Amino-4-methyl-1-phenyl-2-pyrazoline (4.4 g) was added to acetic anhydride (4 ml) at room temperature, and the mixture was stirred for 1 hour. Water was added, the insoluble oil was extracted into chloroform, and the extract was washed with sodium bicarbonate solution and with water, dried (magnesium sulphate) and evaporated to leave a viscous oil. This oil was stirred with methanol to give 3-di-acetylamino-4-methyl-1-phenyl-2-pyrazoline which crystallised from methanol as colourless needles.

The methanol filtrate was evaporated to give a second crop of crystals which, after further recrystallisation from benzene light petroleum (b.p. 60—80) and then methanol, gave 3-acetamido-4-methyl-1-phenyl-2-pyrazoline as colourless prisms, m.p. 150—151°.

Examples 27 and 28

In accordance with the method described in Example 25 there were prepared the following:

Reference Example 27: 3-Acetamido-5-methyl-1-phenyl-2-pyrazoline m.p. 134—136°
Example 28: 3-Butyramido-5-methyl-1-phenyl-2-pyrazoline m.p. 73—75°

Example 29

Preparation of 3-Acetamido-1-(3-trifluoromethylphenyl)-2-pyrazoline

3-Amino-1-(3-trifluoromethylphenyl)-2-pyrazoline (1,0 g) was added to acetic anhydride (5 ml) at room temperature with stirring. The temperature of the mixture rose to 29°. After 20 minutes the mixture was warmed to 37° for 5 minutes and then cooled to produce a semi-solid mass. Water was added and the mixture warmed to 35° to decompose the excess anhydride. The solid product was filtered off and washed with water. The residue was stirred with methanol and the insoluble solid was collected and dried *in vacuo* to give 3-acetamido-1-(3-trifluoromethylphenyl)-2-pyrazoline (600 mg) m.p. 239,8°.

Example 30

Preparation of 3-Butyramido-1-(3-trifluoromethylphenyl)-2-pyrazoline

Butyric anhydride (0,5 ml) was added to a stirred solution of 3-amino-1-(3-trifluoromethylphenyl)-2-pyrazoline (400 mg) in chloroform (5 ml). The resulting mixture was heated to reflux for 1 hour and then evaporated *in vacuo*. The residue was ground up with light petroleum (b.p. 80—100°) to produce pure 3-butyramido-1-(3-trifluoromethylphenyl)-2-pyrazoline (310 mg) m.p. 161—162°.

Example 31

Ointment

| | |
|---|---|
| Active Ingredient | 1.0 g |
| White Soft paraffin | to 100.0 g |

Disperse the Active Ingredient in a small volume of the vehicle. Gradually incorporate this into the bulk to produce a smooth, homogeneous product. Fill into collapsible metal tubes.

Example 32
Cream for Topical Use

| | |
|---|---|
| Active Ingredient | 1,0 g |
| Polawax GP 200 | 20,0 g |
| Lanolin Anhydrous | 2,0 g |
| White Beeswax | 2,5 g |
| Methyl Hydroxybenzoate | 0,1 g |
| Distilled Water | to 100,0 g |

Heat the Polawax, beeswax and lanolin together at 60°. Add a solution of Methyl Hydroxybenzoate Homogenise using high speed stirring. Allow the temperature to fall to 50°. Add and disperse the active ingredient. Allow to cool with slow speed stirring.

Example 33
Lotion for Topical Use

| | |
|---|---|
| Active Ingredient | 1,0 g |
| Sorbitan Monolaurate | 0,6 g |
| Polysorbate 20 | 0,6 g |
| Cetostearyl Alcohol | 1,2 g |
| Glycerin | 6,0 g |
| Methyl Hydroxybenzoate | 0,2 g |
| Purified Water B.P. | to 100,00 ml |

The Methyl Hydroxybenzoate and Glycerin were dissolved in 70 ml of the water at 75°C. The Sorbitan Monolaurate, Polysorbate 20 and Cetostearyl Alcohol were melted together at 75°C and added to the aqueous solution. The resulting emulsion was homogenised, allowed to cool with continuous stirring and the Active Ingredient added as a suspension in the remaining Water. The whole was stirred until homogenised.

Example 34
Eye Drops

| | |
|---|---|
| Compound of Example 17 | 0,5 g |
| Methyl Hydroxybenzoate | 0,01 g |
| Propyl Hydroxybenzoate | 0,04 g |
| Purified Water B.P. | to 100,00 ml |

The Methyl and Propyl Hydroxybenzoates were dissolved in 70 ml Purified Water at 75° and the resulting solution then allowed to cool. The compound of Example 17 was then added and the solution made up to 100 ml with purified water. The solution was sterilised by filtration through a membrane filter 0,22 µm pore size and packed aseptically into suitable sterile containers.

Example 35
Inhibition of Lipoxygenase and Cyclo-oxygenase

In an enzyme assay according to the method of P. Borgeat and B. Samuelsson (J. Biol. Chem., *254*: 2643 (1979)), certain compounds were tested over a wide range of concentrations. The results are shown in Table 1.

TABLE 1
Inhibition of Rabbit Leukocyte Lipoxygenase and Cyclo-oxygenase

| Compound | $IC_{50}$ (μM) Lipoxygenase | $IC_{50}$ (μM) Cyclo-oxygenase |
|---|---|---|
| Example 1 | 1—3 | 1—5 |
| Example 14 | 10 | 30 |
| Example 19 | | |
| Indomethacin | 30 | 0.3 |
| 3-Amino-1-methyl-5-(*p*-chlorophenyl)-2-pyrazoline (Example 2 of UK Patent No. 1 294 035) | 60 | 45 |

Example 36
Comparison of Therapeutic Index of Topical and Systemic Administration

The Therapeutic Index in the rat of the compound of Example 1 upon oral administration was compared with its Therapeutic Index upon intradermal administration.

A. *Oral*

The anti-inflammatory activity of the compound upon oral administration was measured according to the method described by Higgs et al in Biochem. Pharmacol. (1979), 28, 1959—1961 (reduction of carrageenin-induced oedema).

The amount of compound required to produce signs of haemolytic activity toxicity (measured in terms of depression of blood haemoglobin levels and reduction in red cell count) was found by orally administering a daily dose of the compound for four days.

B. *Intradermal*

The anti-inflammatory activity of the compound upon intradermal administration was measured by finding how much of the compound was required to reverse arachidonic acid-induced plasma exudation in the rat skin according to the method of T. J. Williams and M. J. Peck in Nature 270, 530, (1977).

The haemolytic toxicity of the compound upon intradermal administration was found by the method described above. The results are given in Table 5.

TABLE 2
Therapeutic Index of Compound of Example 1 in the Rat

| Route of Administration | Anti-inflammatory Activity | Haemolytic Toxicity | Therapeutic Index |
|---|---|---|---|
| Intradermal | 2 μg per rat | No effect seen at 2,5 mg per rat | >1250 |
| Oral | 50 mg/kg | 11 mg/kg | <5 |

Example 37
Topical Treatment of Ocular Inflammation

Adult albino rabbits weighing 2—3 kg were used.

*Shigella* endotoxin (1 μl) dissolved in sterile saline (20 μl) was injected into the vitreous body of one eye using a 30 gauge needle and an Agla micrometer syringe, contralateral eyes received equal volume of sterile saline. Immediately after injection of endotoxin, eyes were treated topically with a constant volume (50 μl) of either a drug solution or vehicle, three times in 24 hours.

24 hours after treatment began, eyes were examined biomicroscopically and photographed using a Photo-slit lamp. Clinical signs of inflammation, for example flare, exudate in the anterior chamber, conjunctival oedema and vasodilatation and iris hyperaemia were assessed and scored as follows:

0 = normal
1 = mild
2 = moderate
3 = severe
4 = very severe

The mean of the sum of assesses scores is described as an Inflammatory Index.

26 to 28 hours after the endotoxin injection, animals were killed with an overdose of sodium pentobarbitone. Immediately afterwards, aqueous humour was aspirated while taking care not to damage the iris. Leukocytes were counted in a haemocytometer and protein content was determined in an aliquot of aqueous humour diluted with heparinized saline.

Preparation of drug solutions

The compound of Example 1 was dissolved either in saline or in phosphate buffer with the help of polysorbate mono-oleate (Tween 80). For the preparation of the latter solution, Tween 80 was added to the compound and was made into a paste. To this paste, approximately 500 µl of 1 mM phosphate buffer at pH 7.5 was added and whirlimixed. Final dilution was made with phosphate buffer to give a solution containing 5% Tween 80. Indomethacin solution was prepared with equimolar concentrations of sodium carbonate in saline. Dexamethasone was dissolved in saline and its concentration in the text refers to its base. Results are given in Table 3 as mean ± s.e.m.

TABLE 3
Topical Treatment of Ocular Inflammation

| Compound | Dose (µmol) | In aqueous humour % control Leukocytes | Protein | Inflammatory Index % control |
|---|---|---|---|---|
| Example In Saline | 0.18 | 112 ± 33.0 (5) | 81.1 ± 20.6 (5) | 91.7 ± 9.0 (5) |
| | 0.46 | 70.6 ± 18.1** (5) | 79.9 ± 2.3 (5) | 92.3 ± 9.7 (5) |
| | 0.93 | 71.0 ± 22.0** (5) | 86.3 ± 13.6 (5) | 71.7 ± 11.6** (5) |
| | 1.87 | 59.8 ± 11.0* (5) | 70.4 ± 15.0** (5) | 73.8 ± 10.2** (5) |
| | 3.74 | 117.0 ± 30.0 (5) | 112.0 ± 11.0 (5) | 92.0 ± 3.3 (5) |
| Example 1 In Tween 80 | 0.23 | 84.0 ± 15.0 (6) | 114.0 ± 7.4 (6) | 104.0 ± 10.0 (6) |
| | 0.46 | 55.0 ± 12.5* (6) | 72.0 ± 3.9* (6) | 81.2 ± 6.5 (6) |
| | 0.93 | 45.7 ± 6.3* (5) | 84.9 ± 5.5 (5) | 72.0 ± 3.7* (5) |
| | 1.87 | 44.9 ± 6.6* (5) | 77.5 ± 8.5* (5) | 69.6 ± 2.2* (5) |
| | 3.74 | 95.4 ± 25.0 (13) | 98.8 ± 6.2 (13) | 99.5 ± 8.1 (13) |

TABLE 3 (continued)

| Compound | Dose (µmol) | In aqueous humour % control Leukocytes | Protein | Inflammatory Index % control |
|---|---|---|---|---|
| Dexa-methasone | 0,031 | 71,0 ± 9,9 (5) | 74,0 ± 6,2 (5) | 66,3 ± 3,6 (5) |
| | 0,063 | 58,5 ± 13,0* (5) | 43,4 ± 11,0* (5) | 64,2 ± 7,5* (5) |
| | 0,127 | 21,2 ± 6,6* (5) | 21,1 ± 5,5* (5) | 42,8 ± 7,7* (5) |
| | 0,254 | 27,0 ± 3,5* (5) | — | 36,8 ± 5,0* (5) |
| Indo-methacin | 0,014 | 104,0 ± 15,2 (12) | 93,0 ± 4,4 (12) | 100,4 ± 8,4 (12) |
| | 0,028 | 100,0 ± 24,0 (6) | 99,8 ± 11,0 (6) | 99,6 ± 10,5 (6) |
| | 0,07 | 70,0 ± 10,4 (10) | 98,5 ± 4,3 (10) | 86,7 ± 8,9 (10) |
| | 0,14 | 91,0 ± 15,0 (11) | 91,5 ± 8,2 (6) | 87,0 ± 12,5 (6) |
| | 0,28 | 84,0 ± 11,2 (11) | 99,0 ± 8,0 (11) | 85,5 ± 8,0 (11) |

** $p < 0,1$
* $p < 0,05$

Example 38

Preparation of 3-propylamido-1-(m-chlorophenyl)-2-pyrazoline

Amino-1-(*m*-chlorophenyl)-2-pyrazoline (Reference Example 4) (9.78 g, 0.05 mole) was dissolved in lithium aluminium hydride dried tetrahydrofuran (150 ml). The mixture was stirred under nitrogen, cooled in an acetone/carbon dioxide bath to −75° and 1.55 N butyl lithium in *n*-hexane (32.5 ml) was added dropwise at −70° to −75°. To the resulting clear solution at −70° was added dropwise ethyl butyrate (5.8 g, 0.05 mole) in lithium aluminium hydride dried tetrahydrofuran (30 ml). The mixture was stirred, allowed to come to room temperature, then poured into water (900 ml). The oil was extracted twice into dichloromethane, washed with 2N-hydrochloric acid (2 × 150 ml) then with water, dried (magnesium sulphate), filtered and evaporated to dryness *in vacuo* (0.05 mm) to yield the title compound 165—167°.

Analysis:

| | | | |
|---|---|---|---|
| Required: | C, 58.76 : | H, 6.07 : | N, 15.81 |
| Found: | C, 58.77 : | H, 6.19 : | N, 15.63 |

## Claims

1. A pharmaceutical formulation characterised in that the formulation is adapted for only topical, to the exclusion of oral and injectable, administration and comprises a heterocyclic compound of formula (I)

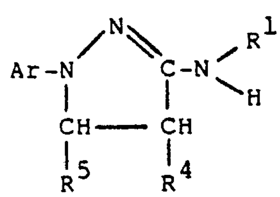

(I)

wherein

Ar is selected from pyridyl or phenyl, each of which may be optionally substituted in one or two positions in the ring by the same or a different substituent, said substituent being selected from trifluoromethyl, fluoro, chloro, bromo and iodo;

$R^1$ is selected from hydrogen and acyl having from 1 to 4 carbon atoms; and

$R^4$ and $R^5$ are the same or different and each is selected from hydrogen and alkyl having from 1 to 4 carbon atoms;

or a pharmaceutically acceptable acid addition salt thereof, together with a pharmaceutically acceptable carrier therefor.

2. A formulation according to claim 1, characterised in that in the compound of formula (I)

Ar is selected from 3-substituted phenyl, 4-substituted phenyl, 3,4-disubstituted phenyl and 5-substituted pyridyl;

$R^1$ is selected from hydrogen and acetyl; and

$R^4$ and $R^5$ are the same or different and each is selected from hydrogen and methyl.

3. A formulation according to claim 1 wherein in the compound of formula (I) Ar is selected from 3- or 4-trifluoromethylphenyl and phenyl substituted by one or more of fluoro, chloro or bromo in the 3-, 4- or 3,4-positions.

4. A formulation according to claim 1 wherein in the compound of formula (I), $R^1$ is hydrogen.

5. A formulation according to claim 2, characterised in that in formula (I)

Ar is selected from phenyl, substituted by one or two substituent(s) selected from 4-fluoro, 4-chloro, 4-bromo and 3-trifluoromethyl, and pyridyl substituted by one substituent selected from 5-chloro, 5-bromo and 5-iodo.

6. A formulation according to claim 5, characterised in that the compound of formula (I) is 3-amino-1-(*m*-trifluoromethylphenyl)-2-pyrazoline.

7. A formulation according to claim 5, characterised in that in formula (I), one of $R^4$ and $R^5$ is methyl.

8. A formulation according to claim 5, characterised in that the compound of formula (I) is selected from

3-amino-1-(*p*-fluorophenyl)-4-methyl-2-pyrazoline; and

3-amino-1-(*p*-fluorophenyl)-5-methyl-2-pyrazoline.

9. A formulation according to any of claims 1 to 8, characterised in that the formulation is in the form of a liniment, lotion, cream, ointment, paste or drops.

10. A formulation according to any of claims 1 to 9, characterised in that the pharmaceutically acceptable carrier comprises a surface active agent.

11. A formulation according to any of claims 1 to 10, characterised in that the formulation is suitable for use in the treatment or prophylaxis of inflammation in a mammal.

**Patentansprüche**

1. Pharmazeutische Formulierung, dadurch gekennzeichnet, daß die Formulierung für die ausschließlich topische, unter Ausschluß der oralen und injizierbaren Verabreichung angepaßt ist, und daß sie eine heterocyclische Verbindung der Formel (I)

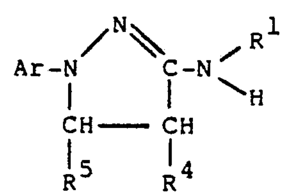

(I)

enthält, worin

Ar aus Pyridyl oder Phenyl ausgewählt ist, wobei jeder der Reste gegebenenfalls in einer oder zwei Position(en) im Ring durch den gleichen oder einen verschiedenen Substituenten substituiert sein kann und wobei der Substituent aus Trifluormethyl, Fluor, Chlor, Brom und Jod ausgewählt ist;

$R^1$ aus Wasserstoff und Acyl mit 1 bis 4 Kohlenstoffatomen ausgewählt ist, und

$R^4$ und $R^5$, die gleich oder verschieden sind, aus Wasserstoff und Alkyl mit 1 bis 4 Kohlenstoffatomen ausgewählt sind;

oder ein pharmazeutisch annehmbares Säureadditionssalz zusammen mit einem pharmazeutisch annehmbaren Träger.

2. Formulierung nach Anspruch 1, dadurch gekennzeichnet, daß in der Verbindung der Formel (I)

Ar aus 3-substituiertem Phenyl, 4-substituiertem Phenyl, 3,4-disubstituierten Phenyl und 5-substituiertem Pyridyl ausgewählt ist;

$R^1$ aus Wasserstoff und Acetyl ausgewählt ist; und

$R^4$ und $R^5$, die gleich oder verschieden sind, jeweils aus Wasserstoff und Methyl ausgewählt sind.

3. Formulierung nach Anspruch 1, worin in der Verbindung der Formel (I) Ar aus 3- oder 4-Trifluormethylphenyl und Phenyl, substituiert durch eines oder mehrere Fluor, Chlor oder Brom in der 3-, 4- oder 3,4-Position, ausgewählt ist.

4. Formulierung nach Anspruch 1, worin in der Verbindung der Formel (I) $R^1$ Wasserstoff ist.

5. Formulierung nach Anspruch 2, dadurch gekennzeichnet, daß in Formel (I) Ar aus Phenyl, substituiert durch einen oder zwei Substituenten, ausgewählt aus 4-Fluor, 4-Chlor, 4-Brom und 3-Trifluormethyl, und Pyridyl, substituiert durch einen Substituenten, ausgewählt aus 5-Chlor, 5-Brom und 5-Jod, ausgewählt ist.

6. Formulierung nach Anspruch 5, dadurch gekennzeichnet, daß die Verbindung der Formel (1) 3-Amino-1-(m-trifluormethylphenyl)-2-pyrazolin ist.

7. Formulierung nach Anspruch 5, dadurch gekennzeichnet, daß in Formel (I) einer der Reste $R^4$ und $R^5$ Methyl ist.

8. Formulierung nach Anspruch 5, dadurch gekennzeichnet, daß die Verbindung der Formel (I) aus 3-Amino-1-(p-fluorphenyl)-4-methyl-2-pyrazolin und 3-Amino-1-(p-fluorphenyl)-5-methyl-2-pyrazolin ausgewählt ist.

9. Formulierung nach einem der Ansprüche 1 bis 8, dadurch gekennzeichnet, daß die Formulierung in Form eines Liniments, einer Lotion, einer Creme, einer Salbe, einer Paste oder in Form von Tropfen vorliegt.

10. Formulierung nach einem der Ansprüche 1 bis 9, dadurch gekennzeichnet, daß der pharmazeutisch annehmbare Träger ein oberflächenaktives Mittel umfaßt.

11. Formulierung nach einem der Ansprüche 1 bis 10, dadurch gekennzeichnet, daß die Formulierung zur Verwendung in der Behandlung oder Prophylaxe eine Entzündung in einem Säugetier geeignet ist.

**Revendications**

1. Composition pharmaceutique, caractérisée en ce qu'elle est adaptée à l'administration par voie topique uniquement, à l'exclusion de la voie orale et de l'injection, et comprend un composé hétérocyclique de formule (I)

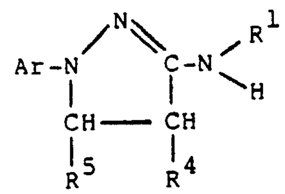

(I)

où Ar est choisi entre pyridyle et phényle, dont chacun peut éventuellement être substitué en une ou deux positions du cycle par un substituant identique ou différent, ce substituant étant choisi entre trifluorométhyle, fluoro, chloro, bromo et iodo;

$R^1$ est choisi entre hydrogène et acyle comptant 1 à 4 atomes de carbone, et

$R^4$ et $R^5$ sont identiques ou différents et choisis chacun entre hydrogène et alcoyle comptant 1 à 4 atomes de carbone;

ou un sel d'addition d'acide pharmaceutiquement acceptable de celui-ci, conjointement avec un excipient pharmaceutiquement acceptable.

2. Composition suivant la revendication 1, caractérisée en ce que dans le composé de formule (I),

Ar est choisi parmi phényle 3-substitué, phényle 4-substitué, phényle 3,4-disubstitué et pyridyle 5-substitué;

$R^1$ est choisi entre hydrogène et acétyle, et

$R^4$ et $R^5$ sont identiques ou différents et sont choisis chacun entre hydrogène et méthyle.

3. Composition suivant la revendication 1, caractérisée en ce que dans le composé de formule (I),

Ar est choisi entre 3- ou 4-trifluorométhylphényle et phényle substitué par un ou plusieurs d'entre fluoro, chloro et bromo aux positions 3, 4 ou 3,4.

4. Composition suivant la revendication 1, caractérisée en ce que dans le composé de formule (I), $R^1$ représente hydrogène.

5. Composittion suivant la revendication 2, caractérisée en ce que dans la formule (I),

Ar est choisi parmi phényle substitué par un ou deux substituants choisis entre 4-fluoro, 4-chloro, 4-bromo et 3-trifluorométhyle, et pyridyle substitué par un substituant choisi entre 5-chloro, 5-bromo et 5-iodo.

6. Composition suivant la revendication 5, caractérisée en ce que le composé de formule (I) est la 3-amino-1-(*m*-trifluorométhylphényl)-2-pyrazoline.

7. Composition suivant la revendication 5, caractérisée en ce que dans la formule (I), l'un d'entre $R^4$ et $R^5$ représente méthyle.

8. Composition suivant la revendication 5, caractérisée en ce que le composé de formule (I) est choisi entre la 3-amino-1-(*p*-fluorophényl)-4-méthyl-2-pyrazoline et la 3-amino-1-(*p*-fluorophényl)-5-méthyl-2-pyrazoline.

9. Composition suivant l'une quelconque des revendications 1 à 8, caractérisée en ce qu'elle est présentée sous la forme d'un liniment, d'une lotion, d'une crème, d'un onguent, d'une pâte ou de gouttes.

10. Composition suivant l'une quelconque des revendications 1 à 9, caractérisée en ce que l'excipient pharmaceutiquement acceptable comprend un agent tensio-actif.

11. Composition suivant l'une quelconque des revendications 1 à 10, caractérisée en ce qu'elle est propre au traitement ou à la prophylaxie d'une inflammation chez un mammifère.